# EUROPEAN PATENT APPLICATION

(11) **EP 3 079 088 A1**
(43) Date of publication of application: **12.10.2016**
(21) Application number: 14873509.5
(22) Date of filing: 18.11.2014
(51) Int. Cl.: G06F 19/00

(54) **USER BEHAVIOR SAFETY MONITORING METHOD AND DEVICE**

(30) Priority: 23.12.2013 CN 201310720012
(71) Applicant: Huawei Technologies Co., Ltd., Longgang District Shenzhen, Guangdong 518129 (CN)
(72) Inventor: WEI, He, Shenzhen Guangdong 518129 (CN); HUANG, Kangmin, Shenzhen Guangdong 518129 (CN)
(74) Representative: Körber, Martin Hans
(86) International application number: PCT/CN2014/091412
(87) International publication number: WO 2015/096567

(57) **Abstract**

The present invention provides a method and device for monitoring user behavior safety, where the method includes: obtaining feature information and a user behavior restriction condition; obtaining physical status information of a user; obtaining a result of user behavior safety assessment according to the feature information, the user behavior restriction condition, and the physical status information of the user; and sending an assessment result indication to the user, where the assessment result indication is used to indicate the result of user behavior safety assessment. According to the present invention, a user can be notified of a result of user behavior safety monitoring in time, avoiding happening of a safety accident.

## Description

### TECHNICAL FIELD

The present invention relates to computer technologies and communications technologies, and in particular, to a method and device for monitoring user behavior safety.

### BACKGROUND

In real life, physical health statuses of many people, such as elder people, patients who have just undergone operations, and chronic alcoholics, are unstable. User behavior safety is at great risk when these people take some activities, for example, when an elder person tries bungee jumping or a patient who has just undergone a heart operation takes a plane. In recent years, with the improvement of people's living standards, increasing attention has been paid to personal health problems. Among measures with respect to personal health, in addition to periodic physical examinations, an increasing quantity of monitoring devices emerge constantly. For example, a portable patient monitor monitors personal physiological parameters, such as blood pressure, blood oxygen, electrocardiograms, respiration, and body temperature, for a long time by using an accompanied collection apparatus that is based on a sensor, playing a role of health protection.

However, unlike general disease diagnosis, determining a personal health status is not only collecting data and analyzing data passively, but also needs to obtain a comprehensive monitoring result according to a specific behavior of a user, for example, an ongoing behavior of the user or a current environment of the user. Currently, a traditional device for monitoring personal health generally relies on passive detection; therefore, timely warning cannot be accomplished.

### SUMMARY

Embodiments of the present invention provide a method and device for monitoring user behavior safety, to implement that a user can be notified of a result of user behavior safety monitoring, avoiding happening of a safety accident.

To resolve the foregoing technical problem, a technical solution provided in the present invention is that: a method for monitoring user behavior safety is provided, where the method includes: obtaining feature information and a user behavior restriction condition, where the feature information includes feature information of a specific location or feature information of a user behavior, and the specific location includes a current location of a user and a target location of the user; and the obtaining feature information and a user behavior restriction condition includes: detecting position information of the user, determining the specific location according to the position information of the user, and obtaining, according to the specific location, the feature information and the user behavior restriction condition that correspond to the specific location; or receiving a position indication sent by a monitoring device in the specific location, and obtaining, according to the position indication, the feature information and the user behavior restriction condition that correspond to the specific location; or receiving position information entered by the user, determining the specific location according to the position information entered by the user, and obtaining, according to the specific location, the feature information and the user behavior restriction condition that correspond to the specific location; obtaining physical status information of the user, where the obtaining physical status information of the user includes: obtaining the physical status information of the user from a prestored historical record; or detecting a current physical status of the user, so as to obtain the physical status information of the user; or receiving the physical status information of the user entered by the user; obtaining a result of user behavior safety assessment according to the feature information, the user behavior restriction condition, and the physical status information of the user; and sending an assessment result indication to the user, where the assessment result indication is used to indicate the result of user behavior safety assessment.

The physical status information of the user includes at least one category of the following information: age, body temperature, blood pressure, heart rate, respiration, medical record, blood element, medical history, occurred abnormal behavior, and medical instruction.

The obtaining a result of user behavior safety assessment according to the feature information, the user behavior restriction condition, and the physical status information of the user includes: obtaining current behavior information of the user according to the feature information and the physical status information of the user; determining, according to the current behavior information of the user, whether a user current behavior meets the user behavior restriction condition; and if the user current behavior meets the user behavior restriction condition, obtaining an assessment result that the user behavior brings no potential safety hazard; or if the user current behavior does not meet the user behavior restriction condition, obtaining an assessment result that the user behavior brings a potential safety hazard.

The assessment result indication includes a first indication and/or a second indication; and the sending an assessment result indication to the user, where the assessment result indication is used to indicate the result of user behavior safety assessment includes: sending the first indication to the user; or sending the second indication to the user; or sending the first indication and the second indication to the user, where the first indication is used to indicate that the result of user behavior safety assessment is that there is no potential safety hazard, and the second indication is used to indicate that the result of user behavior safety assessment is that there is a potential safety hazard. The assessment result indication includes at least one type of indication in the following types: image, audio, vibration, temperature, smell, and indicator.

To resolve the foregoing technical problem, another technical solution provided in the present invention is that: a method for monitoring user behavior safety is provided, where the method includes: obtaining feature information and a user behavior restriction condition, where the feature information includes feature information of a specific location or feature information of a user behavior, and the specific location includes a current location of a user and a target location of the user; obtaining physical status information of the user; obtaining a result of user behavior safety assessment according to the feature information, the user behavior restriction condition, and the physical status information of the user; and sending an assessment result indication to the user, where the assessment result indication is used to indicate the result of user behavior safety assessment.

The obtaining feature information and a user behavior restriction condition includes: detecting position information of the user, determining the specific location according to the position information of the user, and obtaining, according to the specific location, the feature information and the user behavior restriction condition that correspond to the specific location; or receiving a position indication sent by a monitoring device in the specific location, and obtaining, according to the position indication, the feature information and the user behavior restriction condition that correspond to the specific location; or receiving position information entered by the user, determining the specific location according to the position information entered by the user, and obtaining, according to the specific location, the feature information and the user behavior restriction condition that correspond to the specific location.

The obtaining physical status information of the user includes: obtaining the physical status information of the user from a prestored historical record; or detecting a current physical status of the user, so as to obtain the physical status information of the user; or receiving the physical status information of the user entered by the user.

The physical status information of the user includes at least one category of the following information: age, body temperature, blood pressure, heart rate, respiration, medical record, blood element, medical history, occurred abnormal behavior, and medical instruction.

The physical status information of the user includes at least one category of the following information: age, body temperature, blood pressure, heart rate, respiration, medical record, blood element, medical history, occurred abnormal behavior, and medical instruction.

The obtaining a result of user behavior safety assessment according to the feature information, the user behavior restriction condition, and the physical status information of the user includes: obtaining current behavior information of the user according to the feature information and the physical status information of the user; determining, according to the current behavior information of the user, whether a user current behavior meets the user behavior restriction condition; and if the user current behavior meets the user behavior restriction condition, obtaining an assessment result that the user behavior brings no potential safety hazard; or if the user current behavior does not meet the user behavior restriction condition, obtaining an assessment result that the user behavior brings a potential safety hazard.

The assessment result indication includes a first indication and/or a second indication; and the sending an assessment result indication to the user, where the assessment result indication is used to indicate the result of user behavior safety assessment includes: sending the first indication to the user; or sending the second indication to the user; or sending the first indication and the second indication to the user, where the first indication is used to indicate that the result of user behavior safety assessment is that there is no potential safety hazard, and the second indication is used to indicate that the result of user behavior safety assessment is that there is a potential safety hazard.

The assessment result indication includes a first indication and/or a second indication; and the sending an assessment result indication to the user, where the assessment result indication is used to indicate the result of user behavior safety assessment includes: sending the first indication to the user; or sending the second indication to the user; or sending the first indication and the second indication to the user, where the first indication is used to indicate that the result of user behavior safety assessment is that there is no potential safety hazard, and the second indication is used to indicate that the result of user behavior safety assessment is that there is a potential safety hazard.

The assessment result indication includes at least one type of indication in the following types: image, audio, vibration, temperature, smell, and indicator.

To resolve the foregoing technical problems, still another technical solution provided in the present invention is that: a device for monitoring user behavior safety is provided, where the device includes: a first processing unit, configured to obtain feature information and a user behavior restriction condition, where the feature information includes feature information of a specific location or feature information of a user behavior, and the specific location includes a current location of a user and a target location of the user; a second processing unit, configured to obtain physical status information of the user; a safety assessment unit, configured to obtain a result of user behavior safety assessment according to the feature information, the user behavior restriction condition, and the physical status information of the user; and a sending unit, configured to send an assessment result indication to the user, where the assessment result indication is used to indicate the result of user behavior safety assessment.

The device further includes: a positioning unit, configured to detect position information of the user, and the first processing unit is specifically configured to determine the specific location according to the position information of the user, and obtain, according to the specific location, the feature information and the user behavior restriction condition that correspond to the specific location; or the device further includes a receiving unit, configured to receive a position indication sent by a monitoring device in the specific location, and the first processing unit is specifically configured to obtain, according to the position indication, the feature information and the user behavior restriction condition that correspond to the specific location; or the receiving unit is configured to receive position information entered by the user, and the first processing unit is specifically configured to determine the specific location according to the position information entered by the user, and obtain, according to the specific location, the feature information and the user behavior restriction condition that correspond to the specific location.

The device further includes: a storage unit, configured to store a historical record, and the second processing unit is specifically configured to obtain the physical status information of the user from the stored historical record; or the device further includes a detection unit, configured to detect a current physical status of the user, so as to obtain the physical status information of the user, and the second processing unit is specifically configured to obtain the physical status information of the user from the detection unit; or the device further includes a receiving unit, configured to receive the physical status information of the user entered by the user, and the second processing unit is specifically configured to obtain the physical status information of the user from the receiving unit.

The physical status information of the user includes at least one category of the following information: age, body temperature, blood pressure, heart rate, respiration, medical record, blood element, medical history, occurred abnormal behavior, and medical instruction.

The physical status information of the user includes at least one category of the following information: age, body temperature, blood pressure, heart rate, respiration, medical record, blood element, medical history, occurred abnormal behavior, and medical instruction.

The safety assessment unit is specifically configured to obtain current behavior information of the user according to the feature information and the physical status information of the user; determine, according to the current behavior information of the user, whether a user current behavior meets the user behavior restriction condition; and if the user current behavior meets the user behavior restriction condition, obtain an assessment result that the user behavior brings no potential safety hazard; or if the user current behavior does not meet the user behavior restriction condition, obtain an assessment result that the user behavior brings a potential safety hazard.

The assessment result indication includes a first indication and/or a second indication; and the sending unit is specifically configured to send the first indication to the user; or send the second indication to the user; or send the first indication and the second indication to the user, where the first indication is used to indicate that the result of user behavior safety assessment is that there is no potential safety hazard, and the second indication is used to indicate that the result of user behavior safety assessment is that there is a potential safety hazard.

The assessment result indication includes at least one type of indication in the following types: image, audio, vibration, temperature, smell, and indicator.

It can be seen from the foregoing technical solutions that the embodiments of the present invention have the following beneficial effects:

User behavior safety is assessed according to the feature information, the user behavior restriction condition, and the physical status information of the user, and the assessment result is provided to the user in time; therefore, a potential safety hazard can be determined and the user can be notified of the potential safety hazard in time, to avoid happening of a safety accident.

### BRIEF DESCRIPTION OF DRAWINGS

To describe the technical solutions in the embodiments of the present invention more clearly, the following briefly introduces the accompanying drawings required for describing the embodiments. Apparently, the accompanying drawings in the following description show merely some embodiments of the present invention, and a person of ordinary skill in the art may still derive other drawings from these accompanying drawings without creative efforts.
FIG. 1 is a schematic diagram of a system to which a method for monitoring user behavior safety according to an embodiment of the present invention is applied;
FIG. 2 is a schematic flowchart of a method for monitoring user behavior safety according to an embodiment of the present invention;
FIG. 3 is a functional block diagram of a device for monitoring user behavior safety according to an embodiment of the present invention; and
FIG. 4 is a schematic structural diagram of a device for monitoring user behavior safety according to an embodiment of the present invention.

### DESCRIPTION OF EMBODIMENTS

To make the technical solutions in the present invention more comprehensible, the following describes the embodiments of the present invention in detail with reference to the accompanying drawings.

It should be clear that the described embodiments are merely some but not all of the embodiments of the present invention. All other embodiments obtained by a person of ordinary skill in the art based on the embodiments of the present invention without creative efforts shall fall within the protection scope of the present invention.

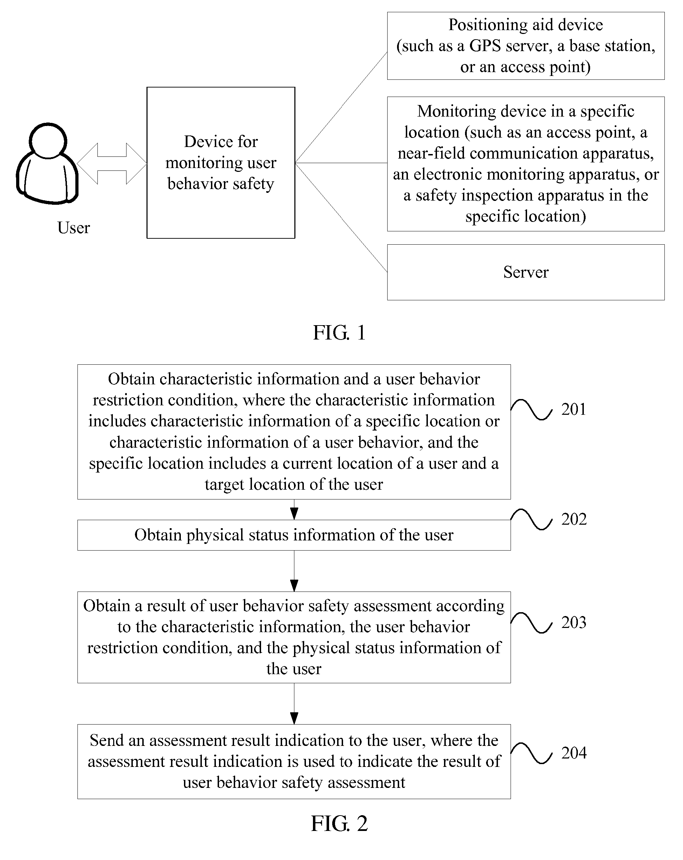

A system used in the technical solutions provided by the embodiments of the present invention is shown in FIG. 1 and mainly includes a device for monitoring user behavior safety and an aid device. A method provided in the embodiments of the present invention is implemented on a side of the device for monitoring user behavior safety and is mainly configured to monitor user behavior safety and provide a result of user behavior safety monitoring to a user in time, where the monitoring user behavior safety and providing a result of user behavior safety monitoring to a user in time herein may be independently completed by the device for monitoring user behavior safety. In the embodiments of the present invention, the device for monitoring user behavior safety may also coordinate with the aid device to implement the monitoring of user behavior safety. The aid device may include a positioning aid device, a monitoring device in a specific location, or a server, where at least one device of the positioning aid device, the monitoring device in the specific location, and the server herein is an optional device. The device for monitoring user behavior safety is a portable device, such as a mobile phone, glasses, a wrist strap, a ring, a next-to-skin chip, or circuit-equipped clothing that is/are held or worn by a user. The positioning aid device may include a global positioning system (Global Positioning System, GPS) server, a base station, or an access point. The monitoring device in the specific location may include an access point, a near field communication apparatus, an electronic monitoring apparatus, a safety inspection apparatus, or the like in the specific location. The device for monitoring user behavior safety provided in the embodiments of the present invention may communicate with the server in a wired manner or in a wireless manner to acquire required information from the server, where a manner of acquiring the information from the server by the device for monitoring user behavior safety includes: periodically acquiring information, acquiring information in a triggered manner, or instantly acquiring information. The server may be located within the specific location, or may be a same physical apparatus as the monitoring device in the specific location.

An embodiment of the present invention provides a method for monitoring user behavior safety. Refer to FIG. 2, which is a schematic flowchart of the method for monitoring user behavior safety according to this embodiment of the present invention. As shown in FIG. 2, the method includes the following steps:
Step 201: Obtain feature information and a user behavior restriction condition, where the feature information includes feature information of a specific location or feature information of a user behavior, and the specific location includes a current location of a user and a target location of the user.

Specifically, a device for monitoring user behavior safety may obtain the feature information and the user behavior restriction condition under the following three trigger conditions:
First: The device for monitoring user behavior safety detects position information of the user; determines the specific location according to the position information of the user, where the specific location includes the current location of the user and the target location of the user, and the target location of the user may be a location at which the user is to arrive; and obtains, according to the determined specific location, the feature information and the user behavior restriction condition that correspond to the specific location. The device for monitoring user behavior safety may interact with a positioning aid device, such as a GPS server, a base station, or an access point, to obtain current position information of the user; and then determine the position information and determine a distance between the position information and a location around, where if the distance is less than a preset threshold, it is deemed that the user is about to arrive at the location.
Second: The device for monitoring user behavior safety receives a position indication sent by a monitoring device in the specific location and obtains, according to the position indication, the feature information and the user behavior restriction condition that correspond to the specific location. When the user is approaching or passing by the monitoring device in the specific location, the monitoring device in the specific location detects the device for monitoring user behavior safety carried by the user and then sends the position indication to the device for monitoring user behavior safety, where the position indication is used to indicate that the user is approaching or passing by the monitoring device in the specific location. Therefore, the device for monitoring user behavior safety may be triggered to obtain the feature information and the user behavior restriction condition. The position indication may be at least one type of indication in the following types: pushed application (Application, App) message, short message service message, and sound wave.
Third: The device for monitoring user behavior safety receives position information entered by the user and determines the specific location according to the position information entered by the user, where the specific location includes a target location of the user; and the device for monitoring user behavior safety obtains, according to the specific location, the feature information and the user behavior restriction condition that correspond to the specific location. The user may enter, on the device for monitoring user behavior safety, position information of the target location; then, the device for monitoring user behavior safety can receive the position information entered by the user, thereby determining the specific location.

In this embodiment of the present invention, optionally, some locations may be preset, and according to the preset locations, screening is performed for the specific location. For example, the preset locations may be those functional locations or special locations, such as an amusement location, a location in a bad environment, and a gymnasium. The screening is performed for the specific location by using the preset locations, where the technical solution provided in this embodiment of the present invention may not be used for a specific location outside a range of the preset locations, and the technical solution provided in this embodiment of the present invention is used only for a specific location within the range of the preset locations; in this way, user behavior safety may be monitored in a targeted manner, avoiding meaningless monitoring of user behavior safety.

In this embodiment of the present invention, the device for monitoring user behavior safety obtains policy information according to a specific location that the user is at or is about to arrive at, and a correspondence between an identifier of a preset specific location and the policy information, where the policy information includes feature information and a user behavior restriction condition that correspond to the specific location, and the feature information includes feature information of the specific location or feature information of a user behavior.

The device for monitoring user behavior safety may obtain locally stored policy information, or may obtain policy information from the server. The device for monitoring user behavior safety may obtain the policy information from the server periodically; or obtain the policy information from the server after being triggered and update the locally stored policy information according to the obtained policy information. The feature information may include the feature information of the specific location or the feature information of the user behavior.

The feature information and the user behavior restriction condition may be determined according to a type of the specific location, a type of the behavior, the restriction condition, a danger level, or the like. For example, as described in Table 1, Table 1 describes examples of the feature information and the user behavior restriction condition:

**Table 1**

| | |
|---|---|
| **Types of specific locations and types of behaviors** | Feature information includes: |
| | 1. Feature information of specific locations: |
| | stimulating amusement location, precision instrument operation room, and steep cliff area |
| | 2. Feature information of user behaviors: |
| | strenuous exercise, nerve-stimulating behavior, long-lasting behavior, body-suppressing behavior, and the like |
| | User behavior restriction conditions include: |
| | no being in a stimulating amusement location; no operating a precision instrument; no being in a steep cliff area; no strenuous exercise; no relatively intense nerve stimulation; no long-time working; and no carrying a load over 50 kilograms. |
| **Restriction conditions** | Feature information includes: |
| | those whose body temperature is higher than 38°C not to enter or participate; those whose recent average heart rate is higher than 90 beats/s not to enter or participate; those whose instant blood pressure is higher than 140/90 mmHg not to enter or participate; those aged over 60 not to enter or participate; those with a history of heart disease not to enter or participate; those having taken neural drugs recently not to enter or participate; those with old bone fractures not to enter or participate; those who faint at the sight of blood not to enter or participate; those carrying a specific virus not to enter or participate; and those having skin damage not to enter or participate |
| | User behavior restriction conditions include: |
| | no strenuous exercise when the body temperature is higher than 40°C; no strenuous exercise when the instant blood pressure is higher than 140/90 mmHg; and no sauna bath when the instant blood pressure is higher than 140/90 mmHg |
| **Danger levels** | Feature information includes: |
| | highly dangerous and slightly dangerous; highly stimulating and fairly stimulating |
| | User behavior restriction conditions include: |
| | no highly stimulating activities |

As described in Table 1, in this embodiment of the present invention, the feature information may be specific to only one category of information in the physical status information of the user, or may be specific to multiple categories of information in the physical status information of the user. For example, the feature information "those whose body temperature is higher than 38°C not to enter or participate" is specific to body temperature in the physical status information of the user.

Step 202: Obtain physical status information of the user.

Specifically, after the device for monitoring user behavior safety obtains the feature information and the user behavior restriction condition, the device for monitoring user behavior safety needs to obtain the physical status information of the user, where the physical status information of the user may be previous physical status information of the user, or may be instant physical status information of the user.

If the physical status information of the user is the previous physical status information of the user, the device for monitoring user behavior safety needs to obtain the physical status information of the user from a prestored historical record.

If the physical status information of the user is the instant physical status information of the user, the device for monitoring user behavior safety may actively detect a current physical status of the user, so as to obtain the physical status information of the user, or the device for monitoring user behavior safety may receive physical status information of the user entered by the user. The device for monitoring user behavior safety may detect the current physical status of the user by using various sensors, such as a temperature sensor, a blood pressure sensor, and a heartbeat sensor.

In this embodiment of the present invention, the physical status information of the user includes at least one category of the following information: age, body temperature, blood pressure, heart rate, respiration, medical record, blood element, medical history, occurred abnormal behavior, and medical instruction.

Step 203: Obtain a result of user behavior safety assessment according to the feature information, the user behavior restriction condition, and the physical status information of the user.

Specifically, the device for monitoring user behavior safety first obtains current behavior information of the user according to the feature information and the physical status information of the user that are obtained; then determines, according to the current behavior information of the user and the user behavior restriction condition, whether a user current behavior meets the user behavior restriction condition; and if it is determined that the user current behavior meets the user behavior restriction condition, obtains an assessment result that the user behavior brings no potential safety hazard, that is, the device for monitoring user behavior safety determines that the result of user behavior safety assessment is that there is no potential safety hazard; or if the user current behavior does not meet the user behavior restriction condition, obtains an assessment result that the user behavior brings a potential safety hazard, that is, the device for monitoring user behavior safety determines that the result of user behavior safety assessment is that there is a potential safety hazard.

For example, as described in Table 2, that the physical status information of the user is body temperature is used as an example to describe a method for obtaining, by the device for monitoring user behavior safety, the result of user behavior safety assessment according to the feature information, the user behavior restriction condition, and the physical status information of the user:

**Table 2**

| **Policy information** | | **Physical status information of the user** | **Result of user behavior safety assessment** |
|---|---|---|---|
| **User behavior restriction condition** | **Feature information** | | |
| No being in a stimulating amusement location | Bungee jumping (stimulating amusement location) | Any | There is a potential safety hazard |
| No being in a stimulating amusement location | Museum | Any | There is no potential safety hazard |
| | Bungee jumping (stimulating amusement location) | Any | There is no potential safety hazard |
| No strenuous exercise when the body temperature is higher than 40°C | Gymnasium | Instant body temperature 41°C | There is a potential safety hazard |
| No strenuous exercise when the body temperature is higher than 40°C | Gymnasium | Instant body temperature 38°C | There is no potential safety hazard |
| No strenuous exercise when the body temperature is higher than 40°C | Music hall | Instant body temperature 41°C | There is no potential safety hazard |
| No strenuous exercise when the body temperature is higher than 40°C | Music hall | Instant body temperature 38°C | There is no potential safety hazard |
| No strenuous exercise when the body temperature is higher than 40°C | Gymnasium, and those whose body temperature is higher than 38°C not to enter or participate | Instant body temperature 41°C | There is a potential safety hazard |
| No strenuous exercise when the body temperature is higher than 40°C | Gymnasium, and those whose body temperature is higher than 38°C not to enter or participate | Instant body temperature 39°C | There is a potential safety hazard |
| No strenuous exercise when the body temperature is higher than 40°C | Gymnasium, and those whose body temperature is higher than 38°C not to enter or participate | Instant body temperature 37°C | There is no potential safety hazard |
| | Those aged over 60 not to enter or participate | 65 years old | There is a potential safety hazard |
| | Those aged over 60 not to enter or participate | 30 years old | There is no potential safety hazard |
| No highly stimulating activities | Highly stimulating | Any | There is a potential safety hazard |
| No highly stimulating activities | Fairly stimulating | Any | There is no potential safety hazard |
| No highly stimulating activities | Slightly dangerous | Any | There is no potential safety hazard |

As described in Table 2, for example, the physical status information of the user is that the instant body temperature is 41°C, the feature information is gymnasium, and the user behavior restriction condition is no strenuous exercise when the body temperature is higher than 40°C. In this case, user current behavior information obtained by the device for monitoring user behavior safety is that a user whose instant body temperature is 41°C is in the gymnasium. The device for monitoring user behavior safety compares the user current behavior information with the user behavior restriction condition, and determines that the user current behavior information does not meet the user behavior restriction condition; therefore, the device for monitoring user behavior safety determines that the result of user behavior safety assessment is that there is a potential safety hazard. For another example, the physical status information of the user is that the instant body temperature is 38°C, the feature information is music hall, and the user behavior restriction condition is no strenuous exercise when the body temperature is higher than 40°C. In this case, user current behavior information obtained by the device for monitoring user behavior safety is that a user whose instant body temperature is 38°C is in the music hall. The device for monitoring user behavior safety compares the user current behavior information with the user behavior restriction condition, and determines that the user current behavior information meets the user behavior restriction condition; therefore, the device for monitoring user behavior safety determines that the result of user behavior safety assessment is that there is no potential safety hazard.

Step 204: Send an assessment result indication to the user, where the assessment result indication is used to indicate the result of user behavior safety assessment.

Specifically, after obtaining the result of user behavior safety assessment, the device for monitoring user behavior safety needs to provide the result of user behavior safety assessment to the user, so as to notify the user of existence of a potential safety hazard and implement timely warning.

In this embodiment of the present invention, a method for providing, by the device for monitoring user behavior safety, the result of user behavior safety assessment to the user may include: sending, by the device for monitoring user behavior safety, an assessment result indication to the user, where the assessment result indication may indicate the result of user behavior safety assessment. In this way, the user may learn, according to the assessment result indication, about whether a current behavior of the user brings a potential safety hazard.

The assessment result indication includes at least one type of indication in the following types: image, audio, vibration, temperature, smell, and indicator.

The assessment result indication includes a first indication and/or a second indication. The sending, by the device for monitoring user behavior safety, an assessment result indication to the user, where the assessment result indication may indicate the result of user behavior safety assessment may be implemented in the following three implementation manners:
First manner: The device for monitoring user behavior safety sends the first indication to the user, where the first indication is used to indicate that the result of user behavior safety assessment is that there is no potential safety hazard. That is, the device for monitoring user behavior safety sends the assessment result indication to the user only when the result of user behavior safety assessment is that there is no potential safety hazard, where the assessment result indication is used to indicate that a user current behavior brings no potential safety hazard; in this case, if the result of user behavior safety assessment is that there is a potential safety hazard, the device for monitoring user behavior safety sends no assessment result indication to the user. For example, the assessment result indication is in a form of an indicator. In this case, the indicator is always on if the result of user behavior safety assessment is always that there is no potential safety hazard; once the result of user behavior safety assessment is that there is a potential safety hazard, the indicator may be off. Therefore, when finding that the indicator is off, the user knows that there is a potential safety hazard.
Second manner: The device for monitoring user behavior safety sends the second indication to the user, where the second indication is used to indicate that the result of user behavior safety assessment is that there is a potential safety hazard. That is, the device for monitoring user behavior safety sends the assessment result indication to the user only when the result of user behavior safety assessment is that there is a potential safety hazard, where the assessment result indication is used to indicate that a user current behavior brings a potential safety hazard. In this case, if the result of user behavior safety assessment is that there is no potential safety hazard, the device for monitoring user behavior safety sends no assessment result indication to the user. For example, the assessment result indication is in a form of audio. In this case, the device for monitoring user behavior safety does not make any sound if the result of user behavior safety assessment is always that there is no potential safety hazard; once the result of user behavior safety assessment is that there is a potential safety hazard, the device for monitoring user behavior safety makes a warning sound. Therefore, when hearing the warning sound made by the device for monitoring user behavior safety, the user knows that the current behavior brings a potential safety hazard.
Third manner: The first indication and the second indication are sent to the user, where the first indication is used to indicate that the result of user behavior safety assessment is that there is no potential safety hazard, and the second indication is used to indicate that the result of user behavior safety assessment is that there is a potential safety hazard. That is, regardless of whether the result of user behavior safety assessment is that there is a potential safety hazard, the device for monitoring user behavior safety sends the assessment result indication to the user, where the assessment result indication is used to indicate whether the user current behavior brings a potential safety hazard. For example, if the first indication is in a form of an image, the device for monitoring user behavior safety displays an image if the result of user behavior safety assessment is that there is no potential safety hazard, and when seeing the image, the user knows that the current behavior brings no potential safety hazard. If the second indication is in a form of audio, the device for monitoring user behavior safety makes a warning sound if the result of user behavior safety assessment is that there is a potential safety hazard, and when hearing the warning sound make by the device for monitoring user behavior safety, the user knows that the current behavior brings a potential safety hazard.

In addition, the term "and/or" in this specification describes only an association relationship for describing associated objects and represents that three relationships may exist. For example, A and/or B may represent the following three cases: only A exists, both A and B exist, and only B exists. In addition, the character "/" in this specification generally indicates an "or" relationship between the associated objects.

Optionally, after sending the assessment result indication to the user, the device for monitoring user behavior safety may further send a notification message to a device associated with a third party, where the notification message carries the result of user behavior safety assessment. For example, a notification message may be sent to a relative or friend of the user, a doctor, a medical organization, or a device or terminal for monitoring user behavior safety of another user nearby, so as to alert the third party to care or treat the user in time.

The embodiments of the present invention further provide apparatus embodiments that implement the steps and the methods in the foregoing method embodiments.

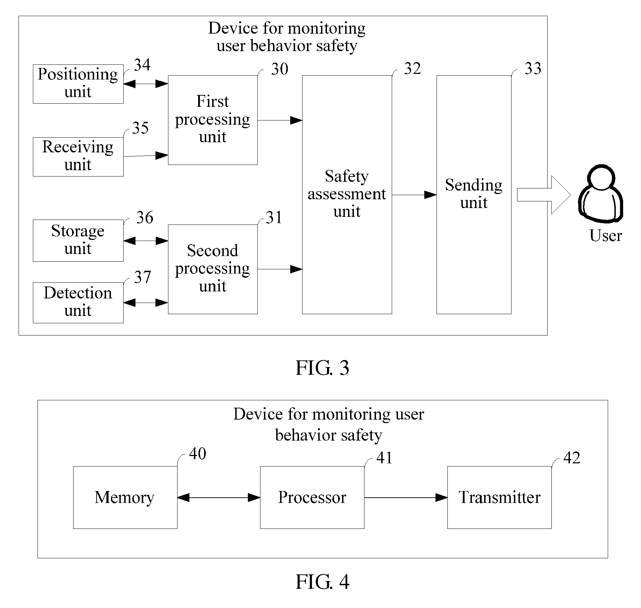

Refer to FIG. 3, which is a functional block diagram of a device for monitoring user behavior safety according to an embodiment of the present invention. As shown in FIG. 3, the device includes:
a first processing unit 30, configured to obtain feature information and a user behavior restriction condition, where the feature information includes feature information of a specific location or feature information of a user behavior, and the specific location includes a current location of a user and a target location of the user;
a second processing unit 31, configured to obtain physical status information of the user;
a safety assessment unit 32, configured to obtain a result of user behavior safety assessment according to the feature information, the user behavior restriction condition, and the physical status information of the user; and
a sending unit 33, configured to send an assessment result indication to the user, where the assessment result indication is used to indicate the result of user behavior safety assessment.

Optionally, the device further includes a positioning unit 34, configured to detect position information of the user, and
the first processing unit 30 is specifically configured to determine the specific location according to the position information of the user, and obtain, according to the specific location, the feature information and the user behavior restriction condition that correspond to the specific location; or
the device further includes a receiving unit 35, configured to receive a position indication sent by a monitoring device in the specific location, and
the first processing unit 30 is specifically configured to obtain, according to the position indication, the feature information and the user behavior restriction condition that correspond to the specific location; or
the receiving unit 35 is configured to receive position information entered by the user, and
the first processing unit 30 is specifically configured to determine the specific location according to the position information entered by the user, and obtain, according to the specific location, the feature information and the user behavior restriction condition that correspond to the specific location.

Optionally, the device further includes a storage unit 36, configured to store a historical record, and
the second processing unit 31 is specifically configured to obtain the physical status information of the user from the stored historical record; or
the device further includes a detection unit 37, configured to detect a current physical status of the user, so as to obtain the physical status information of the user, and
the second processing unit 31 is specifically configured to obtain the physical status information of the user from the detection unit; or
the device further includes a receiving unit 35, configured to receive the physical status information of the user entered by the user, and
the second processing unit 31 is specifically configured to obtain the physical status information of the user from the receiving unit 35.

Optionally, the physical status information of the user includes at least one category of the following information: age, body temperature, blood pressure, heart rate, respiration, medical record, blood element, medical history, occurred abnormal behavior, and medical instruction.

Optionally, the safety assessment unit 32 is specifically configured to:
obtain current behavior information of the user according to the feature information and the physical status information of the user;
determine, according to the current behavior information of the user, whether a user current behavior meets the user behavior restriction condition; and
if the user current behavior meets the user behavior restriction condition, obtain an assessment result that the user behavior brings no potential safety hazard; or if the user current behavior does not meet the user behavior restriction condition, obtain an assessment result that the user behavior brings a potential safety hazard.

Optionally, the assessment result indication includes a first indication and/or a second indication; and the sending unit 33 is specifically configured to:
send the first indication to the user; or
send the second indication to the user; or
send the first indication and the second indication to the user, where
the first indication is used to indicate that the result of user behavior safety assessment is that there is no potential safety hazard, and the second indication is used to indicate that the result of user behavior safety assessment is that there is a potential safety hazard.

Optionally, the assessment result indication includes at least one type of indication in the following types: image, audio, vibration, temperature, smell, and indicator.

It should be noted that in actual application, the sending unit 33 may be a display screen, a generating apparatus, a vibration apparatus, or the like. A communication manner that can be supported by the receiving unit 35 includes at least one of the following manners: wired communication, wireless communication, and near field communication. The detection unit 37 may include at least one sensor, and the sensor is used to detect the physical status information of the user. The positioning unit 34 may interact with a GPS server, a base station, or an access point, to obtain the position information of the user; and provide user information to the first processing unit 30.

Refer to FIG. 4, which is a schematic structural diagram of a device for monitoring user behavior safety according to an embodiment of the present invention. As shown in FIG. 4, the device includes:
a memory 40, configured to store one group of or more groups of program code; and
a processor 41, coupled with both the memory 40 and a transmitter 42 separately and configured to invoke the program code stored in the memory 40, so as to execute the following method shown in FIG. 2, which specifically includes: obtaining feature information and a user behavior restriction condition, where the feature information includes feature information of a specific location or feature information of a user behavior, and the specific location includes a current location of a user and a target location of the user; obtaining physical status information of the user; and obtaining a result of user behavior safety assessment according to the feature information, the user behavior restriction condition, and the physical status information of the user, where
the transmitter 42 is configured to send an assessment result indication to the user, where the assessment result indication is used to indicate the result of user behavior safety assessment.

Because the processor in this embodiment can execute the method shown in FIG. 2, for a portion that is not described in detail in this embodiment, reference may be made to related descriptions about FIG. 2.

The technical solutions in the embodiments of the present invention have the following beneficial effects:
1. User behavior safety is assessed according to the feature information, the user behavior restriction condition, and the physical status information of the user, and the assessment result is provided to the user in time; therefore, a potential safety hazard can be determined and the user can be notified of the potential safety hazard in time, to avoid happening of a safety accident.
2. User behavior safety is assessed according to one or more categories of physical status information of the user; therefore, user behavior safety can be assessed comprehensively and accuracy of user behavior safety monitoring is improved.
3. The physical status information of the user may be previous physical status information of the user. Compared with the method in a conventional technical solution where only current physical status information of a user is referenced, the technical solutions of the present invention obtain a monitoring result that is more reasonable, more comprehensive, and more accurate, and are particularly applicable to a user who is currently in a good physical status but has a medical history.
4. The device for monitoring user behavior safety is a portable device; therefore the device is convenient for the user to carry and more flexible in application and may be applied to varied scenarios.
5. The device for monitoring user behavior safety can independently monitor user behavior safety without interacting with an external device. Therefore, the device is more flexible and convenient in application and may be applied to varied scenarios, improving processing efficiency of the monitoring device; in addition, the device has a good independent processing capability and high stability, better helping protection of personal privacy.

The foregoing descriptions are merely exemplary embodiments of the present invention, but are not intended to limit the present invention. Any modification, equivalent replacement, or improvement made without departing from the spirit and principle of the present invention should fall within the protection scope of the present invention.

## Claims

1. A method for monitoring user behavior safety, wherein the method comprises:
obtaining feature information and a user behavior restriction condition, wherein the feature information comprises feature information of a specific location or feature information of a user behavior, and the specific location comprises a current location of a user and a target location of the user; and the obtaining feature information and a user behavior restriction condition comprises:
detecting position information of the user, determining the specific location according to the position information of the user, and obtaining, according to the specific location, the feature information and the user behavior restriction condition that correspond to the specific location; or receiving a position indication sent by a monitoring device in the specific location, and obtaining, according to the position indication, the feature information and the user behavior restriction condition that correspond to the specific location; or receiving position information entered by the user, determining the specific location according to the position information entered by the user, and obtaining, according to the specific location, the feature information and the user behavior restriction condition that correspond to the specific location;
obtaining physical status information of the user, wherein the obtaining physical status information of the user comprises: obtaining the physical status information of the user from a prestored historical record; or detecting a current physical status of the user, so as to obtain the physical status information of the user; or receiving the physical status information of the user entered by the user;
obtaining a result of user behavior safety assessment according to the feature information, the user behavior restriction condition, and the physical status information of the user; and
sending an assessment result indication to the user, wherein the assessment result indication is used to indicate the result of user behavior safety assessment.

2. The method according to claim 1, wherein the physical status information of the user comprises at least one category of the following information: age, body temperature, blood pressure, heart rate, respiration, medical record, blood element, medical history, occurred abnormal behavior, and medical instruction.

3. The method according to claim 1, wherein the obtaining a result of user behavior safety assessment according to the feature information, the user behavior restriction condition, and the physical status information of the user comprises:
obtaining current behavior information of the user according to the feature information and the physical status information of the user;
determining, according to the current behavior information of the user, whether a user current behavior meets the user behavior restriction condition; and
if the user current behavior meets the user behavior restriction condition, obtaining an assessment result that the user behavior brings no potential safety hazard; or if the user current behavior does not meet the user behavior restriction condition, obtaining an assessment result that the user behavior brings a potential safety hazard.

4. The method according to claim 1, wherein the assessment result indication comprises a first indication and/or a second indication; and the sending an assessment result indication to the user, wherein the assessment result indication is used to indicate the result of user behavior safety assessment comprises:
sending the first indication to the user; or
sending the second indication to the user; or
sending the first indication and the second indication to the user, wherein
the first indication is used to indicate that the result of user behavior safety assessment is that there is no potential safety hazard, and the second indication is used to indicate that the result of user behavior safety assessment is that there is a potential safety hazard.

5. The method according to claim 1, wherein the assessment result indication comprises at least one type of indication in the following types: image, audio, vibration, temperature, smell, and indicator.

6. A method for monitoring user behavior safety, wherein the method comprises:
obtaining feature information and a user behavior restriction condition, wherein the feature information comprises feature information of a specific location or feature information of a user behavior, and the specific location comprises a current location of a user and a target location of the user;
obtaining physical status information of the user;
obtaining a result of user behavior safety assessment according to the feature information, the user behavior restriction condition, and the physical status information of the user; and
sending an assessment result indication to the user, wherein the assessment result indication is used to indicate the result of user behavior safety assessment.

7. The method according to claim 6, wherein the obtaining feature information and a user behavior restriction condition comprises:
detecting position information of the user,
determining the specific location according to the position information of the user, and
obtaining, according to the specific location, the feature information and the user behavior restriction condition that correspond to the specific location; or
receiving a position indication sent by a monitoring device in the specific location, and
obtaining, according to the position indication, the feature information and the user behavior restriction condition that correspond to the specific location; or
receiving position information entered by the user,
determining the specific location according to the position information entered by the user, and
obtaining, according to the specific location, the feature information and the user behavior restriction condition that correspond to the specific location.

8. The method according to claim 6, wherein the obtaining physical status information of the user comprises:
obtaining the physical status information of the user from a prestored historical record; or
detecting a current physical status of the user, so as to obtain the physical status information of the user; or
receiving the physical status information of the user entered by the user.

9. The method according to claim 6, wherein the physical status information of the user comprises at least one category of the following information: age, body temperature, blood pressure, heart rate, respiration, medical record, blood element, medical history, occurred abnormal behavior, and medical instruction.

10. The method according to claim 8, wherein the physical status information of the user comprises at least one category of the following information: age, body temperature, blood pressure, heart rate, respiration, medical record, blood element, medical history, occurred abnormal behavior, and medical instruction.

11. The method according to claim 6, wherein the obtaining a result of user behavior safety assessment according to the feature information, the user behavior restriction condition, and the physical status information of the user comprises:
obtaining current behavior information of the user according to the feature information and the physical status information of the user;
determining, according to the current behavior information of the user, whether a user current behavior meets the user behavior restriction condition; and
if the user current behavior meets the user behavior restriction condition, obtaining an assessment result that the user behavior brings no potential safety hazard; or if the user current behavior does not meet the user behavior restriction condition, obtaining an assessment result that the user behavior brings a potential safety hazard.

12. The method according to claim 6, wherein the assessment result indication comprises a first indication and/or a second indication; and the sending an assessment result indication to the user, wherein the assessment result indication is used to indicate the result of user behavior safety assessment comprises:
sending the first indication to the user; or
sending the second indication to the user; or
sending the first indication and the second indication to the user, wherein
the first indication is used to indicate that the result of user behavior safety assessment is that there is no potential safety hazard, and the second indication is used to indicate that the result of user behavior safety assessment is that there is a potential safety hazard.

13. The method according to claim 11, wherein the assessment result indication comprises a first indication and/or a second indication; and the sending an assessment result indication to the user, wherein the assessment result indication is used to indicate the result of user behavior safety assessment comprises:
sending the first indication to the user; or
sending the second indication to the user; or
sending the first indication and the second indication to the user, wherein
the first indication is used to indicate that the result of user behavior safety assessment is that there is no potential safety hazard, and the second indication is used to indicate that the result of user behavior safety assessment is that there is a potential safety hazard.

14. The method according to claim 6, wherein the assessment result indication comprises at least one type of indication in the following types: image, audio, vibration, temperature, smell, and indicator.

15. A device for monitoring user behavior safety, wherein the device comprises:
a first processing unit, configured to obtain feature information and a user behavior restriction condition, wherein the feature information comprises feature information of a specific location or feature information of a user behavior, and the specific location comprises a current location of a user and a target location of the user;
a second processing unit, configured to obtain physical status information of the user;
a safety assessment unit, configured to obtain a result of user behavior safety assessment according to the feature information, the user behavior restriction condition, and the physical status information of the user; and
a sending unit, configured to send an assessment result indication to the user, wherein the assessment result indication is used to indicate the result of user behavior safety assessment.

16. The device according to claim 15, wherein
the device further comprises a positioning unit, configured to detect position information of the user, and
the first processing unit is specifically configured to determine the specific location according to the position information of the user, and obtain, according to the specific location, the feature information and the user behavior restriction condition that correspond to the specific location; or
the device further comprises a receiving unit, configured to receive a position indication sent by a monitoring device in the specific location, and
the first processing unit is specifically configured to obtain, according to the position indication, the feature information and the user behavior restriction condition that correspond to the specific location; or
the receiving unit is configured to receive position information entered by the user, and
the first processing unit is specifically configured to determine the specific location according to the position information entered by the user, and obtain, according to the specific location, the feature information and the user behavior restriction condition that correspond to the specific location.

17. The device according to claim 15, wherein
the device further comprises a storage unit, configured to store a historical record, and
the second processing unit is specifically configured to obtain the physical status information of the user from the stored historical record; or
the device further comprises a detection unit, configured to detect a current physical status of the user, so as to obtain the physical status information of the user, and
the second processing unit is specifically configured to obtain the physical status information of the user from the detection unit; or
the device further comprises a receiving unit, configured to receive the physical status information of the user entered by the user, and
the second processing unit is specifically configured to obtain the physical status information of the user from the receiving unit.

18. The device according to claim 15, wherein the physical status information of the user comprises at least one category of the following information: age, body temperature, blood pressure, heart rate, respiration, medical record, blood element, medical history, occurred abnormal behavior, and medical instruction.

19. The device according to claim 17, wherein the physical status information of the user comprises at least one category of the following information: age, body temperature, blood pressure, heart rate, respiration, medical record, blood element, medical history, occurred abnormal behavior, and medical instruction.

20. The device according to claim 15, wherein the safety assessment unit is specifically configured to:
obtain current behavior information of the user according to the feature information and the physical status information of the user;
determine, according to the current behavior information of the user, whether a user current behavior meets the user behavior restriction condition; and
if the user current behavior meets the user behavior restriction condition, obtain an assessment result that the user behavior brings no potential safety hazard; or if the user current behavior does not meet the user behavior restriction condition, obtain an assessment result that the user behavior brings a potential safety hazard.

21. The device according to claim 15, wherein the assessment result indication comprises a first indication and/or a second indication; and the sending unit is specifically configured to:
send the first indication to the user; or
send the second indication to the user; or
send the first indication and the second indication to the user, wherein
the first indication is used to indicate that the result of user behavior safety assessment is that there is no potential safety hazard, and the second indication is used to indicate that the result of user behavior safety assessment is that there is a potential safety hazard.

22. The device according to claim 15, wherein the assessment result indication comprises at least one type of indication in the following types: image, audio, vibration, temperature, smell, and indicator.
